Europäisches Patentamt

European Patent Office

Office européen des brevets

(19)

(11) Veröffentlichungsnummer: **0 238 952 B1**

(12) **EUROPÄISCHE PATENTSCHRIFT**

(45) Veröffentlichungstag der Patentschrift: 27.03.91

(51) Int. Cl.5: **A61M 25/00, A61B 17/34**

(21) Anmeldenummer: **87103703.2**

(22) Anmeldetag: **13.03.87**

(54) **Katheter für die perkutane Gastrostomie.**

(30) Priorität: **27.03.86 DE 3610419**

(43) Veröffentlichungstag der Anmeldung:
**30.09.87 Patentblatt 87/40**

(45) Bekanntmachung des Hinweises auf die
Patenterteilung:
**27.03.91 Patentblatt 91/13**

(84) Benannte Vertragsstaaten:
**AT DE ES FR GB IT SE**

(56) Entgegenhaltungen:
**WO-A-83/00631**        **GB-A- 2 146 902**
**GB-A- 2 150 837**      **US-A- 3 539 034**
**US-A- 3 592 184**      **US-A- 4 169 464**
**US-A- 4 608 965**

(73) Patentinhaber: **Pfrimmer-Viggo GmbH + Co.
KG**
**Langemarckplatz 3 Postfach 28 80**
**W-8520 Erlangen(DE)**

(72) Erfinder: **Iwatschenko, Peter**
**Bürgerholzweg 4**
**W-8524 Neunkirchen(DE)**

(74) Vertreter: **Wuesthoff, Franz, Dr.-Ing. et al
Wuesthoff & Wuesthoff Patent- und Rechtsanwälte Schweigerstrasse 2
W-8000 München 90(DE)**

## Beschreibung

Die Erfindung betrifft einen Katheter aus Kunststoff für die perkutane Gastrostomie mit den Merkmalen des Oberbegriffes des Patentanspruches 1.

Ein derartiger Katheter ist aus der Zeitschrift "Die Medizinische Welt", 1985, 36, S. 1297-1301, bekannt.

Bei der perkutanen Gastrostomie wird der Patient unter Umgehung seiner Kau- und Schluckorgane derart "künstlich ernährt", daß ihm die Diäten direkt in den Magen zugeführt werden.

Für die direkte Alimentation in den Magen ist die sogenannte Witzel-Fistel bekannt. Ein Witzel-Kanal ist aber operativ sehr aufwendig und wurde deshalb in jüngerer Zeit zunehmend durch die perkutane endoskopisch kontrollierte Gastrostomie ersetzt, welche neben die bekannten Applikationsformen mittels einer transnasalen Ernährungssonde und die Katheter-Jejunostomie tritt.

Die perkutane, endoskopisch kontrollierte Gastrostomie (s. z.B. "Die Medizinische Welt", 1985, 36, S. 1297-1301) benutzt einen Katheter aus Polyurethan, der mittels eines Gastroskopes (Endoskop) und eines durch die Bauchdecke und die Magenwand eingeführten Fadens im Magen positioniert wird.

Das Einführen des Katheters bei der perkutanen, endoskopisch kontrollierten Gastrostomie gemäß dem Stand der Technik verlangt einen erheblichen apparativen Aufwand sowie einiges Geschick des Operateurs. Zunächst wird ein Gastroskop in den Magen eingeführt und der Magen durch Luftinsufflation entfaltet. Im Bereich des Lichtscheines wird durch Druck mit dem Finger auf die Bauchdecken die Vorwölbung der Magenschleimhaut endoskopisch sichtbar. An einer geeigneten Stelle wird sodann eine Kanüle so weit vorgeschoben, bis sie mittels des Gastroskopes im Magenlumen erkennbar wird. Ein langer Führungsfaden wird sodann durch die Kanüle in das Magenlumen eingeführt und dort mittels der Biopsie-Zange erfaßt und aus dem Mund des Patienten herausgezogen. Mittels des Führungsfadens wird dann in umgekehrter Richtung der Katheter durch den Mund des Patienten hindurch in den Magen und aus ihm heraus durch die Bauchdecke gezogen.

Ein wesentliches Problem bei der perkutanen Gastrostomie besteht darin, daß der Katheter sicher im Magen positioniert sein muß. Insbesondere beim Einführen des Katheters ergeben sich diesbezüglich Probleme, weil der Magen sich nach der Insufflation wieder zusammenzieht, so daß der Katheter Gefahr läuft, aus der Magenwand herauszurutschen, was fatale Folgen hat. Bei der bekannten, endoskopisch kontrollierten Gastrostomie wird deshalb am Katheter eine Silikonscheibe angebracht, die an der Mageninnenwand anliegt und somit dafür sorgt, daß das magenseitige Ende des Katheters in bezug auf die Magenwand fest positioniert bleibt und nicht aus dem Magen herausrutschen kann.

Aus der GB-A-2 146 902 ist ein Katheter bekannt, bei dem sich kein Abschnitt nach dem Einführen in die Körperhöhle spiralförmig krümmt. Die Spitze des Katheters kann direkt an der Wand der Körperhöhle anliegen.

Aus der WO-A-83 00 631 ist ein Katheter bekannt, bei dem zwei gegenüberliegende Einlagen in der Wandung vorgesehen sind. Diese Einlagen dienen aber dazu, eine möglichst stabile gestreckte Form des Schlauches zu erzielen, also keine Krümmung.

Der Erfindung liegt die Aufgabe zugrunde, ein Katheter der gattungsgemäßen Art bereitzustellen, welches in relativ einfacher Weise im Magen positionierbar ist, ohne daß eine Gefahr des Herausrutschens des Katheters aus dem Magen besteht.

Erfindungsgemäße Lösungen der Aufgabe sind in den Patentansprüchen 1, 4, 6 und 7 gekennzeichnet.

Vorteilhafte Ausgestaltungen der Erfindung sind in den abhängigen Ansprüchen beschrieben.

Statt der beim Stand der Technik bekannten Silikonscheibe, deren Einbringung in den Magen offensichtlich Schwierigkeiten bereiten kann, sieht die Erfindung vor, daß der Katheter derart ausgestaltet ist, daß er nach dem Einführen in das Magenlumen dort selbst für seine feste Positionierung sorgt, indem er sich derart krümmt, daß er nicht mehr aus dem Magen herausrutschen kann.

In einer bevorzugten Ausgestaltung der Erfindung ist vorgesehen, daß der Katheter auf einem in ihn eingeschobenen Trokar eine geradlinig gestreckte Form annimmt (so daß der Katheter mit eingeschobenem Trokar durch die Bauchdecken und die Magenwand in das Magenlumen einführbar ist) und nach dem Herausziehen des Trokars aus dem Katheter in einem Abschnitt selbsttätig eine gekrümmte Form annimmt, die sich von innen an die Magenwand anlegt und derart ein Herausrutschen des Katheters aus dem Magen ausschließt.

In einer bevorzugten Weiterbildung der Erfindung ist vorgesehen, daß sich der genannte Abschnitt des Katheters im Magen spiralförmig krümmt, wobei die Spiralform bevorzugt einen sogenannten Unterzug in Richtung auf das Mageninnere aufweist. Diese Ausgestaltung des Katheters mit einem spiralförmigen Abschnitt einschließlich eines Unterzuges hat den Vorteil, daß Zugkräfte am Katheter gleichmäßig über einen größeren Teil des gekrümmten Abschnittes verteilt werden.

In einer anderen Variante der Erfindung ist vorgesehen, daß in der Wandung des Katheters zumindest eine Einlage aus einer sogenannten "Memory"-Legierung eingebettet ist, welche bei einer vorgegebenen Temperatur einen bestimmten Abschnitt des Katheters gestreckt hält und bei einer anderen vorgegebenen Temperatur diesen Abschnitt in eine gekrümmte, z.B. spiralförmige Gestalt bringt.

Es ist auch möglich, das letztgenannte Verfahren der Krümmung des vorgegebenen Katheterabschnittes mittels einer Einlage aus einer "Memory"-Legierung mit dem erstgenannten Verfahren zu kombinieren, bei dem der Katheter derart vorgeformt wird, daß er ohne Belastung von selbst die gekrümmte Form annimmt, wenn kein Trokar in ihn eingeschoben ist.

In einer weiteren Variante der Erfindung ist vorgesehen, daß der Katheter als Arretierungseinrichtung zumindest ein schirmartig aufklappbares Bauteil aufweist. Das aufklappbare Bauteil liegt zunächst eng an der Katheterwandung an (steht also nicht senkrecht von dieser ab), faltet sich aber nach dem Einführen des Katheters durch die Bauchdecke und die Magenwand in das Magenlumen selbsttätig auf, so daß es an der Mageninnenwand anliegt und somit das magenseitige Ende des Katheters im Magenlumen sichert.

Die schirmartig am Katheter aufklappbaren Bauteile sind in einer bevorzugten Ausgestaltung der Erfindung mittels einer im Körper auflösbaren Abdeckung, wie Gelatine, zunächst beim Einführen des Katheters in den Magen in Anlage an dem Katheter gehalten. Nach dem Einführen in das Magenlumen löst sich die Gelatine auf, so daß die federgespannten Bauteile aufklappen und sich an die Mageninnenwand anlegen.

In einer anderen Variante der Erfindung können die aufklappbaren Bauteile auch mittels einer Einlage aus einer "Memory"Legierung aufspannbar sein. Einzelheiten über den "Memory"Effekt bei Legierungen finden sich beispielsweise in der Veröffentlichung "Technische Mitteilung Krupp-Forschungsberichte", Band 34 (1976), Heft 1 (Aufsatz von F. Baumgart, J. Jorde und H. Reiß).

Nachfolgend werden Ausführungsbeispiele der Erfindung anhand der Zeichnung näher beschrieben. Dabei zeigt bzw. zeigen:

Fig. 1,    2 und 3 schematisch die Verwendung des erfindungsgemäßen Katheters;

Fig. 4     einen Teil des Katheters im Detail;

Fig. 5     einen Schnitt durch den Katheter im Bereich des krümmbaren Abschnittes;

Fig. 6     ein weiteres Ausführungsbeispieles eines erfindungsgemäßen Katheters; und

Fig. 7     den in Fig. 6 gezeigten Katheter in aufgeklapptem Zustand.

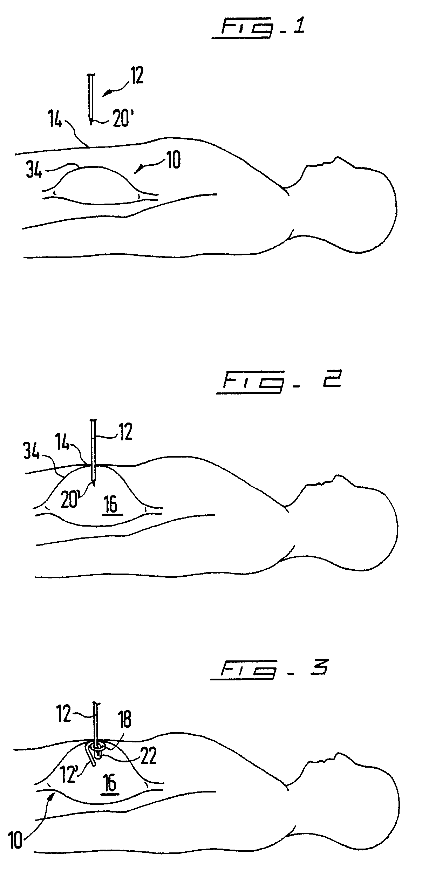

Die Fig. 1, 2 und 3 zeigen einen mittels perkutaner Gastrostomie zu ernährenden Patienten, in dessen Magen 10 der Katheter 12 direkt durch die Bauchdecke 14 eingeführt werden soll.

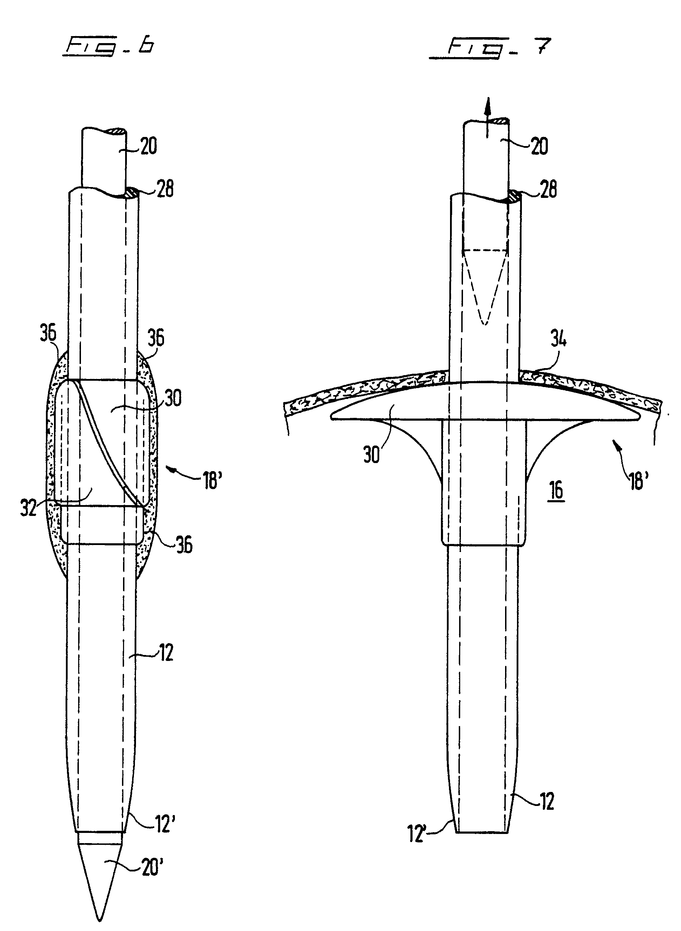

In den Katheter 12 aus Kunststoff ist zunächst ein Trokar (s. a. Fig. 4 und 6) eingeschoben, welcher den Katheter 12 geradlinig streckt. Der Trokar 20 dichtet mit seiner Spitze 20' das untere Ende des Katheters 12 ab und wird mitsamt dem Katheter 12 durch die Bauchdecke 14 und die Magenwand 34 hindurch in das Magenlumen 16 eingeführt. Zuvor wurde dem Patienten Natriumbicarbonat und Ascorbinsäure eingegeben, so daß sich im Magen $CO_2$ bildet. Die gebildeten Gase blähen den Magen auf, welcher sich gemäß Fig. 2 entfaltet, wobei sich die Magenwand 34 der Bauchdecke 14 nähert. An geeigneter Stelle wird der Trokar mit dem aufgeschobenen Katheter 12 durch die Bauchdecke 14 und die Magenwand 34 hindurch in das Magenlumen 16 eingeschoben (Fig. 2).

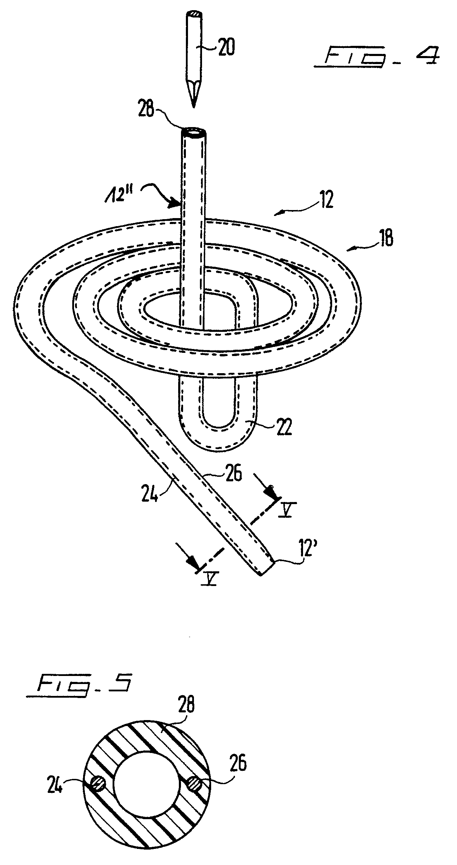

Sodann wird der Trokar 20, dessen Spitze mit dem Bezugszeichen 20' versehen ist, aus dem Katheter 12 herausgezogen. Der Kunststoff-Katheter 12 ist so vorgeformt, daß er sich nach Herausziehen des Trokars sofort gemäß Fig. 3 in einem Abschnitt 18 spiralförmig krümmt.

Der gekrümmte Abschnitt 18 des Katheters 12 legt sich innenseitig an die Magenwand 34, so daß das Ende 12' des Katheters im Magenlumen 16 positioniert ist und nicht aus dem Magen 10 herausrutschen kann.

Fig. 4 zeigt den gemäß Fig. 3 im Magen 10 gehaltenen Abschnitt des Katheters 12 in vergrößertem Maßstab. Sobald der Trokar 20 aus dem Katheter 12 gezogen ist, nimmt dieser in einem vorgegebenen Abschnitt 18 die gezeigte Spiralform an. Diese selbsttätige Krümmung des Katheters 12 kann zum einen dadurch erfolgen, daß der Katheter selbst im Abschnitt 18 spiralförmig vorgeformt ist und nur bei eingeschobenem Trokar 20 geradlinig gestreckt wird, und/oder daß Einlagen 24, 26 in der Wandung 28 des Katheters 12 vorgesehen sind, die sich im Abschnitt 18 nach Herausziehen des Trokars spiralförmig winden. Hierzu sind die Einlagen 24, 26 aus einer "Memory"-Legierung hergestellt, die zunächst bei normaler Zimmertemperatur weitgehend eine geradlinig, gestreckte Form aufweisen, während sie bei Körpertemperatur (37° C) im gewünschten Abschnitt 18 des Katheters die in Fig. 4 gezeigte Spiralform annehmen.

Gemäß den Fig. 3 und 4 ist der spiralförmige Abschnitt 18 des Katheters 12 mit einem sogenannten Unterzug 22 versehen, welcher in das Mageninnere gerichtet ist. Dieser Unterzug gewährleistet beim Auftreten von Zugkräften am Katheter 12 eine Verteilung der Druckkräfte der Mageninnenwand auf mindestens eine Windung des spiralförmigen Abschnittes 18, so daß das Ende 12' des Katheters nicht aus dem Magen 10 herausrutschen kann.

Die Fig. 6 und 7 zeigen ein weiters Ausführungsbeispiel eines Katheters 12, dessen Ende 12' sicher im Magen 10 positionierbar ist. Das Einführen des Katheters 12 mittels eines Trokars 20 erfolgt genau so wie es anhand der Fig. 1-3 vorstehend beschrieben ist. Anstelle der selbsttätigen Krümmung des Katheters 12 mittels Vorspannung im Material oder einer Einlage aus einer "Memory"-Legierung ist beim Ausführungsbeispiel gemäß den Fig. 6 und 7 eine Anordnung aus im Magen schirmartig aufklappbaren Bauteilen 30, 32 vorgesehen. Zunächst werden die aufklappbaren Bauteile 30, 32 gemäß Fig. 6 in flacher Anlage an den Katheter 12 gehalten. Hierzu sind sie von einer Gelatine-Abdeckung 36 aus physiologisch verträglichen Alkoholen umgeben, die mit dem Katheter 12 in das Magenlumen 16 eingeführt wird. Dort löst sich die Gelatine-Abdeckung 36 auf, so daß die aufklappbaren Bauteile 30, 32 unter Federspannung die in Fig. 7 gezeigte, aufgeklappte Stellung annehmen. Es versteht sich, daß die aufklappbaren Bauteile 30, 32 fest mit dem Katheter 12 verbunden sind. Gemäß Fig. 7 legen sich die aufgeklappten Bauteile 30, 32 wie ein Regenschirm innenseitig an die Magenwand 34, so daß das Ende 12' des Katheters 12 nicht aus dem Magen 10 herausrutschen kann.

Die erforderliche Federspannung zum Aufklappen der Bauteile 30, 32 kann auch durch eine Einlage aus einer "Memory"-Legierung erzeugt werden.

Außenseitig der Bachdecke 14 kann der Katheter mit einer herkömmlichen Kunststoffscheibe gesichert werden, die sich von außen an die Bauchdecke anlegt.

Der gekrümmt ausgebildete Katheter gemäß dem in den Fig. 1-4 gezeigten Ausführungsbeispiel läßt sich einfach durch Herausd rehen aus dem Magen entfernen. Bei dem anderen Ausführungsbeispiel gemäß den Fig. 6 und 7 sind die Federkräfte so bemessen, daß die aufklappbaren Bauteile bei extrem starkem Zug am Katheter nachgeben.

## Ansprüche

1. Katheter aus Kunststoff für die perkutane Gastrostomie zum Einführen in den Magen mit einem Abschnitt (18), der beim Einführen des Katheters (12) geradlinig gestreckt ist und nach dem Einführen eine gekrümmte Form annimmt, dadurch **gekennzeichnet,** daß die gekrümmte Form des Abschnittes (18) spiralförmig ist derart, daß das Auslaufende (12') des Katheters auf einer Seite von der Ebene der Spirale absteht und daß auf der anderen Seite der Spiralebene ein Einlaufabschnitt (12'') des Katheters etwa senkrecht zur Spiralebene steht.

2. Katheter nach Anspruch 1, dadurch **gekennzeichnet,** daß auf der einen Seite der Spiralebene ein Unterzug (22) vorgesehen ist.

3. Katheter nach einem der Ansprüche 1 oder 2, dadurch **gekennzeichnet,** daß der Katheter auf einem in ihn eingeschobenen Trokar (20) eine geradlinig gestreckte Form annimmt und nach dem Herausziehen des Trokars (20) in dem genannten Abschnitt (18) selbsttätig die gekrümmte Form annimmt.

4. Katheter aus Kunststoff für die perkutane Gastrostomie zum Einführen in den Magen mit einem Abschnitt (18), der beim Einführen des Katheters (12) geradlinig gestreckt ist und nach dem Einführen eine gekrümmte Form einnimmt, dadurch **gekennzeichnet,** daß in der Wandung (28) des Katheters (12) zumindest eine Einlage (24, 26) aus einer sogenannten "Memory"-Legierung eingebettet ist, welche bei einer vorgegebenen Temperatur den genannten Abschnitt (18) des Katheters (12) gestreckt läßt und bei einer anderen vorgegebenen Temperatur die gekrümmte Form annimmt.

5. Katheter nach Anspruch 4, dadurch **gekennzeichnet,** daß zwei Einlagen (24, 26) diametral gegenüberliegend in die Katheter-Wandung (28) eingebettet sind.

6. Katheter aus Kunststoff für die perkutane Gastrostomie zum unststofflzum Einführen in den Magen mit einem Abschnitt (18'), der nach dem Einführen des Katheters schirmartig aufklappbar ist, dadurch **gekennzeichnet,** daß das schirmartig aufklappbare Bauteil (30, 32) vor dem Einführen mittels einer Abdeckung (36) aus durch Körpersäfte lösbarem Material, wie Gelatine, in Anlage an dem Katheter (12) gehalten wird.

7. Katheter aus Kunststoff für die perkutane Gastrostomie zum Einführen in den Magen mit einer Arretierungseinrichtung, die in dem Magen ihre Form ändert, mit einem schirmartig aufklappbaren Bauteil, dadurch **gekennzeichnet,** daß das schirmartig aufklappbare Bauteil (30, 32) mittels einer Einlage aus einer "Memory"-Legierung aufspannbar ist.

8. Katheter nach einem der Ansprüche 6 oder 7,
dadurch **gekennzeichnet,**
daß zumindest ein aufklappbares Bauteil federgespannt ist.

## Claims

1. A catheter of plastic material for percutaneous gastrostomy to be inserted in the stomach, comprising a portion (18) which is stretched out rectilinearly during introduction of the catheter and assumes a curved configuration upon insertion, **characterized** in that the curved configuration of the portion (18) is spiral, that the outlet end (12') of the catheter projects out of the plane of the spiral at one side, and that an inlet portion (12") of the catheter extends approximately vertically to the plane of the spiral at the other side thereof.

2. The catheter as claimed in claim 1, characterized in that a bearing portion (22) is provided at one side of the plane of the spiral.

3. The catheter as claimed in one of claims 1 or 2, characterized in that the catheter assumes a rectilinear stretched configuration on a trocar (20) which is slipped inside the catheter and, upon withdrawal of the trocar (20), automatically assumes the curved configuration in the said portion (18).

4. A catheter of plastic material for percutaneous gastrostomy to be inserted in the stomach, comprising a portion (18) which is stretched out rectilinearly during introduction of the catheter and assumes a curved configuration upon insertion, **characterized** in that at least one laid-in member (24,26) made of a so-called memory alloy is embedded in the wall (28) of the catheter (12) and leaves the portion (18) of the catheter (12) stretched at one given temperature, whereas it will adopt the curved configuration at another given temperature.

5. The catheter as claimed in claim 4, characterized in that two laid-in members (24,26) are embedded diametrically opposite each other in the catheter wall (28).

6. A catheter of plastic material for percutaneous gastrostomy to be inserted in the stomach, comprising a portion (18') which is unfoldable like an umbrella upon insertion of the catheter, **characterised** in that, prior to the introduction, the structural component (30,32) adapted to be opened like an umbrella is held in engagement with the catheter (12) by a cover (36) made of material which is soluble by the humors of the body, such as gelatin.

7. A catheter of plastic material for percutaneous gastrostomy to be inserted in the stomach, comprising a check means which changes in shape inside the stomach, including a structural component which is unfoldable like an umbrella, **characterized** in that the structural component (30,32) adapted to be opened like an umbrella is unfoldable by a laid-in member made of a memory alloy.

8. The catheter as claimed in one of claims 6 or 7, characterized in that at least one component adapted to be opened is spring loaded.

## Revendications

1. Cathéter en matière plastique pour la gastrotomie percutanée à introduire dans l'estomac avec une partie (18), qui est étendue linéairement lors de l'introduction du cathéter (12) et qui prend une forme incurvée après l'introduction,
caractérisé en ce que,
la forme incurvée de la partie (18) est en forme de spirale, de sorte que l'extrémité terminale (12') du cathéter s'étend d'un côté du plan de la spirale et que de l'autre côté du plan de la spirale une partie d'entrée (12") du cathéter se trouve à peu près perpendiculaire au plan de la spirale.

2. Cathéter selon la revendication 1,
caractérisé en ce qu'on a prévu un soutien (22) d'un côté du plan de la spirale.

3. Cathéter selon l'une des revendications 1 ou 2,
caractérisé en ce que
le cathéter prend une forme d'extension linéaire, sur un trocart inséré en lui et qu'après extraction du trocart (20) il prend de lui-même dans la partie citée (18) la forme incurvée.

4. Cathéter en matière plastique pour la gastrotomie percutanée à introduire dans l'estomac avec une partie (18), qui est étendue linéairement lors de l'introduction du cathéter (12) et qui prend une forme incurvée après l'introduction,
caractérisé en ce qu'on incorpore dans la paroi (28) du cathéter (12) au moins une couche (24, 26) de ce qu'on appelle un alliage à mémoire qui laisse étendue linéairement pour une température prescrite la partie citée (18) du cathéter (12) et qui prend la forme incurvée pour l'autre température prescrite.

5. Cathéter selon la revendication 4, caractérisé en ce qu'on incorpore deux couches (24, 26) diamétralement opposées, dans la paroi du cathéter (28).

6. Cathéter en matière plastique pour la gastrotomie percutanée à introduire dans l'estomac avec une partie (18), qui après l'introduction du cathéter est dépliable comme un parapluie, caractérisé en ce que, la pièce constitutive (30, 32) déployable comme un parapluie est appliquée au contact du cathéter (12), avant l'introduction, au moyen de matériau soluble dans les sucs digestifs, tel que la gélatine.

7. Cathéter en matière plastique pour la gastrotomie percutanée à introduire dans l'estomac, avec un dispositif de blocage, qui modifie sa forme dans l'estomac, avec une pièce constitutive déployable en forme de parapluie, caractérisé en ce que, la pièce constitutive déployable en forme de parapluie (30, 32) peut être mise sous contrainte au moyen de l'incorporation d'un alliage à mémoire.

8. Cathéter selon l'une des revendications 6 ou 7, caractérisé en ce qu'au moins une pièce constitutive déployable est soumise à la contrainte d'un ressort.

FIG - 1

FIG - 2

FIG - 3

FIG.4

FIG.5

FIG.6

FIG.7